# EUROPÄISCHE PATENTSCHRIFT

(11) **EP 2 121 044 B1**
(45) Veröffentlichungstag und Bekanntmachung des Hinweises auf die Patenterteilung: **29.05.2013**
(21) Anmeldenummer: 07818332.4
(22) Anmeldetag: 21.09.2007
(51) Int. Cl.: A61L 2/18, A61L 29/14, A61L 29/16

(54) **ZUSAMMENSETZUNG, INSBESONDERE FÜR DAS BLOCKIEREN VON KATHETERN**
COMPOSITION, PARTICULARLY FOR BLOCKING CATHETERS
COMPOSITION, EN PARTICULIER POUR LE BLOCAGE DES CATHÉTERS

(30) Priorität: 23.12.2006 DE 102006062111
(43) Veröffentlichungstag der Anmeldung: 25.11.2009
(73) Patentinhaber: Farco-Pharma GmbH, 50670 Köln (DE)
(72) Erfinder: VESTWEBER, Anna-Maria, 51399 Burscheid (DE); MEIER, Andreas, 13629 Berlin (DE)
(74) Vertreter: Von Rohr
(86) Internationale Anmeldenummer: PCT/EP2007/008242
(87) Internationale Veröffentlichungsnummer: WO 2008/077441

(56) Entgegenhaltungen:
- EP-A- 0 220 890
- EP-A- 0 455 475
- EP-A- 0 937 394
- WO-A-01/76548
- WO-A-02/05188
- WO-A-03/006071
- US-A- 5 145 667
- US-A1- 2002 051 797
- US-A1- 2002 125 462
- US-A1- 2003 144 362
- US-A1- 2005 215 978
- JONES, G. LL. ET AL: "Effect of triclosan on the development of bacterial biofilms by urinary tract pathogens on urinary catheters ." JOURNAL OF ANTIMICROBIAL CHEMOTHERAPY, VOL. 57, NO. 2, PP. 266-272. CODEN: JACHDX. ISSN: 0305-7453., Februar 2006 (2006-02), XP002472794
- JONES G L ET AL: "Inhibition of crystalline Proteus mirabilis biofilm formation on urinary catheters by triclosan." ABSTRACTS OF THE GENERAL MEETING OF THE AMERICAN SOCIETY FOR MICROBIOLOGY, Bd. 103, 2003, Seiten Q-151 URL, XP009096912 & 103RD AMERICAN SOCIETY FOR MICROBIOLOGY GENERAL MEETING; WASHINGTON, DC, USA; MAY 18-22, 2003 ISSN: 1060-2011
- SKAARE A B ET AL: "Does the nature of the solvent affect the anti-inflammatory capacity of triclosan ? An experimental study" JOURNAL OF CLINICAL PERIODONTOLOGY, VOL. 24, NO. 2, PP. 124-8. JOURNAL CODE: 0425123. ISSN: 0303-6979., Februar 1997 (1997-02), XP009096918
- KJAERHEIM V (REPRINT) ET AL: "ORGANIC-SOLVENTS AND OILS AS VEHICLES FOR TRICLOSAN IN MOUTHRINSES - A CLINICAL-STUDY" SCANDINAVIAN JOURNAL OF DENTAL RESEARCH, VOL. 102, NO. 5, PP. 306-308. ISSN: 0029-845X. PB - MUNKSGAARD INT PUBL LTD, 35 NORRE SOGADE, PO BOX 2148, DK-1016 COPENHAGEN, DENMARK., Oktober 1994 (1994-10), XP009096917

## Beschreibung

Die vorliegende Erfindung betrifft eine Vorrichtung zur Katheterisierung, insbesondere zur Katheterisierung der Harnblase, enthaltend eine Zusammensetzung, insbesondere eine pharmazeutische Zusammensetzung, in Form einer flüssigen Zubereitung, wobei die Zusammensetzung - in Kombination und jeweils in wirksamen, insbesondere pharmazeutisch wirksamen Mengen - (a) Triclosan und (b) mindestens ein Polyol enthält. Die erfindungsgemäß vorgesehene Zusammensetzung eignet sich vorzugsweise zur Verwendung als Blockiermittel (Blockungsmittel) für Katheter, insbesondere Harnblasenkatheter, wie sie beispielsweise im Rahmen einer Langzeitkatheterisierung eingesetzt werden.

In der alltäglichen Praxis gehören - trotz insgesamt verbesserter Therapieoptionen - Patienten mit einer sogenannten Dauerableitung - d. h. Patienten mit einer sogenannten Langzeitkatheterisierung der Harnblase, bei welcher ein Harnblasenkatheter für einen längeren Zeitraum beispielsweise zur dauerhaften Ableitung von Urin oder zur regelmäßigen Spülung der Harnblase mit beispielsweise pharmazeutisch wirksamen Zusammensetzungen eingesetzt ist - zu einer großen Patientengruppe.

Die Katheterisierung eines Patienten erfolgt mit einem sogenannten Blasenkatheter, welcher je nach Einsatzart als Einmal- oder Dauerkatheter ausgebildet sein kann. Dauerkatheter sind insbesondere in bezug auf eine Langzeitkatheterisierung konzipiert. Sie weisen häufig an ihrer Spitze bzw. an dem in die Harnblase einzubringenden Endbereich eine Erweiterungsvorrichtung, insbesondere einen sogenannten Ballon, auf, welcher beispielsweise durch das Befüllen mit einer Flüssigkeit dilatiert bzw. aufgefüllt und somit sozusagen "aufgeblasen" werden kann. Das Auffüllen der Erweiterungsvorrichtung erfolgt dabei insbesondere nach Applikation des Katheters in die Blase, nämlich wenn die Spitze bzw. der Endbereich des Katheters in das Lumen der Harnblase eingeführt worden ist. Durch das Dilatieren bzw. Auffüllen der Erweiterungsvorrichtung wird der Katheter in der Harnblase fixiert, da die vergrößerte Erweiterungsvorrichtung nicht mehr durch den schmalen Harnleiter abgeführt werden kann. Hierdurch sind Dauerkatheter selbsthaltend bzw. selbstfixierend. Mit anderen Worten wird der Katheter somit in der Harnblase gewissermaßen blockiert bzw. geblockt.

Dauerkatheter, welche eine derartige Erweiterungsvorrichtung aufweisen, werden im allgemeinen auch als sogenannte Ballonkatheter bezeichnet. Neben den unterschiedlichen Formen Ihrer Spitze und der Anzahl der Öffnungen unterscheidet man hier zusätzlich in Zweiwege- und Dreiwegekatheter. Diesbezüglich weist der Zweiwegekatheter einen Kanal bzw. Schlauch insbesondere zur Harnableitung und einen Kanal insbesondere zum Befüllen der Erweiterungsvorrichtung des Katheters auf. Bei einem Dreiwegekatheter liegt neben den oben genannten Kanälen bzw. Schläuchen noch ein dritter Kanal bzw. Schlauch vor, welcher zusätzlich zur Einbringung von Spüllösungen genutzt werden kann. Um ein ungewünschtes Entweichen der zum Befüllen der Erweiterungsvorrichtung verwendeten Flüssigkeit zu vermeiden, ist der Befüllungskanal des Katheters häufig mit einem Ventil bzw. mit einer Verschlußvorrichtung ausgestattet.

Hinsichtlich der Applikation von Blasenkathetern kann zwischen einer transurethalen Applikation, bei welcher der Katheter durch die Harnröhre in die Harnblase eingeführt wird, und einer suprapubischen Applikation differenziert werden. Bei der suprapubischen Applikation wird der Katheter durch die Bauchdecke invasiv oberhalb des Schambeines in die Harnblase eingebracht. Für beide Applikationsformen werden im allgemeinen speziell an die jeweilige Applikationsform angepaßte Dauerkatheter verwendet.

Im Rahmen einer Dauer- bzw. Langzeitkatheterisierung von Patienten unter Verwendung von transurethalen oder suprapubischen Verweil- bzw. Langzeitkathetern treten häufig Komplikationen auf, wie die Entstehung von Harnwegsinfekten und sogenannten Inkrustationen insbesondere an der Katheteroberfläche sowohl in bezug auf der dem Gewebe zugewandten Oberfläche als auch in bezug auf das Lumen bzw. die Innenseite des zur Harnableitung dienenden Schlauches des Katheters.

Bei den Inkrustationen handelt es sich - wie nachfolgend noch erörtert - um insbesondere bakteriell verursachte Ablagerungen, welche einerseits das sogenannte Katheterauge - also die zur Harnblase orientierte Öffnung des Schlauches - bzw. das Lumen des zur Harnableitung vorgesehenen Schlauches verstopfen können. Derartige Inkrustationen bzw. Ablagerungen führen insbesondere beim Entfernen des Katheters durch Traumatisierung der Harnröhrenschleimhaut zu schmerzhaften Irritationen. Zudem ist durch die Verstopfung des Schlauchsystems eine einwandfreie Funktion des Katheters häufig nicht gewährleistet.

Derartige Katheterverstopfungen treten bei etwa 40 bis 50 % der Dauerkatheterträger auf. Derartige Verstopfungserscheinungen sind für den Patienten mit großen Nachteilen verbunden. So verursachen durch Inkrustationen hervorgerufene Katheterverstopfungen beim Patienten häufig Schmerzen insbesondere durch Harnverhalt, Streß, Angst sowie Inkontinenz durch den Katheter umgehenden Urin. Zudem zieht der Wechsel eines verstopften Katheters häufig die Ausbildung von narbigen Veränderungen bzw. Harnröhrenverletzungen nach sich, wodurch Harnwegsinfektionen begünstigt werden. Eine Katheterneuanlage bzw. -applikation gestaltet sich dann häufig schwierig.

Ohne sich auf eine bestimmte Theorie beschränken zu wollen, wird die Inkrustation von Kathetern häufig durch eine Infektion mit insbesondere grammnegativen Keimen verursacht, welche das Enzym Urease synthetisieren. Dabei verläuft die Inkrustationsbildung stufenweise ab: Den ersten wichtigen Schritt stellt die Adhäsion von Bakterien am Kathetermaterial dar. Hierdurch entsteht zunächst ein Bakterienfilm, welcher zudem Zelldetritus und Albumin enthält. Zu den zur Inkrustation wesentlich beitragenden Bakterien zählt beispielsweise *Proteus mirabilis.* Diese besitzen ein sehr hohes Haftpotential auch auf sehr glatten Oberflächen. Die vorgenannten Bakterien sowie eine große Anzahl weiterer Bakterien sind in der Lage, das Enzym Urease zu bilden, welches eine entscheidende Rolle bei der Entstehung von Inkrustationen spielt.

Die nachfolgende Auflistung gibt an, welche Bakterienstämme besonders zur Ureasebildung neigen, wobei die in Klammern angegebenen Prozentwerte den Anteil ureasepositiver Kulturen angeben:

| | |
|---|---|
| Proteusstämme | (92 bis 99 %), |
| Providentiastämme | (97 bis 99 %), |
| *Klebsiella pneumoniae* | (64 %), |
| *Pseudomonas aeroginosa* | (93 %), |
| Serratiastämme | (12 bis 99 %), |
| *Enterobacter aerogenes* | (3%), |
| *Escherichia coli* | (0 %), |
| Staphylokokken. | |

Der Wirkmechanismus der Urease kann dahingehend veranschaulicht werden, daß dieses Enzym, welches die biochemische Bezeichnung Harnstoffamidohydrolase trägt, den Abbau von Harnstoff zu Kohlendioxid und Wasser katalysiert, wobei zunächst Carbamidsäure und Ammoniak entstehen. Es erfolgt ein spontaner Zerfall der Carbamidsäure in Kohlendioxid und Ammoniak.

In wäßriger Lösung bilden sich dann aus den beiden Endprodukten, nämlich Kohlendioxid und Ammoniak, Ammoniumionen und Bicarbonat (Hydrogencarbonat), was zu einer deutlichen Alkalisierung des Urins führt. Hierdurch resultiert eine übersättigte Lösung an Struvit und Hydroxylapatit. Daraus entstehende Kristallisationskeime können dann im Biofilm integriert werden oder aber primär in diesem selbst entstehen.

Das resultierende Kristallwachstum bzw. die Kristallaggregationen stellt die zweite Stufe des Inkrustationsprozesses dar, wobei die Bakterien in das Inkrustat eingelagert werden und so für Antibiotika und antimikrobiellen Spüllösungen nicht mehr vollständig zugänglich sind.

Vor diesem Hintergrund sind im Stand der Technik zahlreiche Versuche bzw. Ansätze entwickelt worden, welche die Bildung von Inkrustationen an Kathetern verringern sollen. Die meisten diesbezüglichen Ansätze konnten im Rahmen von klinischen Studien aber nicht manifestiert werden:
Ein erster Ansatz besteht darin, inkrustationshemmende Kathetermaterialien zu entwickeln. Diesbezüglich konnten jedoch Hydrogel- bzw. Silberbeschichtungen von Kathetern bei Langzeitträgern eine Besiedelung mit ureasebildenden Keimen mit nachfolgender Inkrustation nicht verhindern. Vergleichbares wurde auch für antiseptische oder antimikrobielle Beschichtungen festgestellt.

Auch bei Silikonkathetern kommt es oftmals zu Störungen. Aufgrund der Fremdkörpereigenschaften bildet der Katheter einen Kristallisationsherd. Die Haftung von Bakterien auf dem Silikonmaterial ist zwar nicht so stark, wie es beispielsweise für Latex der Fall ist, eine Inkrustation kann jedoch allenfalls verzögert, jedoch nicht vermieden werden. Weiterhin kann es aufgrund der verminderten Haftfähigkeit am Kathetermaterial zu Verschleppung von Sedimenten in die Harnblase kommen, wo derartige Verschleppungen dann als Impfkristalle für die Blasensteinentstehung wirken können.

Antimikrobielle Katheterspüllösungen sowie eine Langzeittherapie mit Antibiotika zeigen ebenfalls keine ausreichende Effektivität. Die in die Inkrustationen eingebetteten Bakterien erweisen sich als weitgehend unempfindlich gegenüber solchen Einflüssen.

Auch orale Therapien mit dem Ziel der Harnansäuerung brachten keinen Erfolg.

Eine weitere Möglichkeit zur Verringerung von Inkrustationen besteht in der Verwendung von Spüllösungen ohne antibiotische Eigenschaften und/oder in der Realisierung relativ kurzer Wechselintervalle in bezug auf den Katheter. Häufige Katheterwechsel sind wegen der negativen Begleiterscheinungen, wie Probleme hinsichtlich der Hygiene, Patiententoleranz und Kosten, jedoch nicht als optimal anzusehen. Spüllösungen ohne antibiotische Eigenschaften sind zudem häufig ineffektiv.

Mit rein mechanisch wirkenden Lösungen, wie einer isotonen Kochsalzlösung, wird außer einem gewissen Ausspülungseffekt keine weitere Wirkung beobachtet. Durch die mit der Spülung erzielte Verdünnung des Urins kann die Inkrustationsbildung nur in begrenztem Umfang reduziert werden. Eine umfassende Prävention oder sogar Beseitigung von Inkrustationen ist hierdurch nicht möglich.

Die Verwendung von Antiseptika zeigt keine generelle Wirkung zur Auflösung bzw. Verhinderung von Inkrustationen. Lediglich eine gewisse indirekte Prävention durch Hemmung des Keimwachstums ist in geringem Umfang möglich. Diesbezüglich konnte bei Spülungen mit Silbernitrat gezeigt werden, daß zwar die Keimzahl reduziert wird, die Behandlung erwies sich jedoch als schmerzhaft. Auch die Verwendung von Chlorhexidin führte nur kurzzeitig zu einer gewissen Keimreduzierung. Bei einem Langzeiteinsatz tritt jedoch keine signifikante Wirkung auf. Chlorhexidin ist zur Hemmung des Bakterienwachstums indiziert, nicht jedoch zur Prävention oder Auflösung von Inkrustationen. Weiterhin ist der Einsatz einer EDTA-Lösung (Ethylendiamintetraessigsäure) physiologisch bedenklich. Eine gewisse Wirkung einer EDTA-Lösung wird bei Konzentrationen von mehr als 10 % sowie pH-Werten oberhalb von 10 erzielt. Jedoch liegt die physiologisch zulässige Konzentration bei etwa 5,5 %, und der pH-Wert des Harns liegt unter 7,5. Daher ist beim Einsatz einer reinen EDTA-Lösung eine kaum zu vertretende langwierige Dauerspülung notwendig.

Weiterhin können grundsätzlich auch azide Verbindungen zur Prävention bzw. Auflösung von Inkrustationen verwendet werden, jedoch führen sie oftmals zu Irritationen der Harnblase bzw. des Harnleiters und sind daher in der Regel insbesondere in bezug auf eine Langzeitanwendung nicht geeignet. In diesem Zusammenhang konnte bei einer zweimal täglich durchgeführte Blasenspülung mit Essigsäure keine signifikante Veränderung der Keimzahl gefunden werden, wobei es zudem zu Harnblasenirritationen gekommen ist. Der Einsatz von Mandelsäure ist nicht zur Auflösung bestehender Inkrustationen geeignet, wobei auch bei dem Einsatz von Mandelsäure häufig starke Beschwerden beobachtet wurden. Spülungen des Katheters mit zitronensäurehaltigen Lösungen können nur in gewissem Maße Harnwegsinfektionen verhindern bzw. in geringem Umfang schon bestehende Harnwegsinfekte günstig beeinflussen. Hinsichtlich der Verwendung von Säuren als Spüllösung ist jedoch problematisch, daß in bezug auf eine gewisse Auflösung der Inkrustation innerhalb eines realistischen Zeitraumes der pH-Wert der Spüllösung permanent bei etwa 4 liegen muß, was jedoch problematisch ist.

Weiterhin ist im Stand der Technik bekannt, in Verbindung mit der Anwendung von Kathetern Substanzen mit antimikrobieller Wirkung, wie Triclosan, einzusetzen:
So beschreibt die WO 02/05188 A1 die Verwendung einer Lösung zur Herstellung eines Materials zur Verwendung in bezug auf einen implantierbaren Katheter, wobei die Lösung einen niederen Alkohol, wie Ethanol, Propanol, Isopropanol oder Butanol, und ein Additiv, wie Triclosan, enthält. Die Entgegenhaltung fokussiert vorrangig auf Katheter, welche beispielsweise zur intravenösen Verabreichung von Substanzen eingesetzt werden. Dabei soll die in diesem Dokument beschriebene Zusammensetzung im Lumen des Katheterschlauches zum Ersatz von Blutflüssigkeit eingesetzt werden. Die Verwendung eines niederen Alkohols ist dabei nicht unproblematisch, insbesondere da derartige Verbindungen das Kathetermaterial übermäßig stark angreifen und auch eine gewisse Toxizität aufweisen - zumal die in diesem Dokument beschriebene Zusammensetzung oftmals in direktem Kontakt mit einer Körperflüssigkeit, wie Blut, steht.

Weiterhin betrifft die EP 0 874 655 B1 bzw. das hieraus hervorgegangene deutsche Patent DE 696 29 128 T2 hydrophil oder hydrophob modifizierte, polymere medizinische Artikel, wie z. B. Katheter, die durch Behandlung mit einer Behandlungslösung erhalten werden, die neben einem hydrophilen oder hydrophoben Polymer eine Kombination von Chlorhexidin und Triclosan enthält. Diese Dokumente betreffen somit eine Kombination von Chlorhexidin und Triclosan.

Die EP 0 830 107 B1 bzw. das hieraus hervorgegangene deutsche Patent DE 696 33 177 T2 betrifft antimikrobisch imprägnierte Katheter und medizinische Implantate sowie entsprechende Imprägnierungsverfahren. Diesbezüglich wird zunächst eine antimikrobische Zusammensetzung mit einem antimikrobischen Agenz in einem organischen Lösemittel hergestellt und anschließend ein durchdringendes Agenz zu der Lösung hinzugefügt, anschließend ein alkalisierendes Agenz zu der Lösung hinzugesetzt und die so resultierende antimikrobische Zusammensetzung zumindest in einem Bereich eines medizinischen Implantats unter Bedingungen, bei denen die antimikrobische Zusammensetzung das Material des medizinischen Implantats durchdringt, angewendet. Das dort beschriebene Verfahren, welches auch die Verwendung von Triclosan umfassen kann, ist jedoch aufwendig und die Dosierung der Wirksubstanz ist oftmals nicht optimal.

Weiterhin betrifft die EP 0 433 332 B1 bzw. das hieraus hervorgegangene deutsche Patent DE 689 24 981 T2 antimikrobische Gegenstände, ihre Herstellung sowie ihren Gebrauch. Das dort beschriebene Verfahren umfaßt die Bildung eines Artikels durch Tauchen einer Form aus nichtvulkanisiertem Naturkautschuklatex, die durch Koagulieren eines alkalischen Naturkautschuklatex gebildet wurde, in ein antimikrobielles Mittel, welches Triclosan sein kann, um - nachdem der Artikel geformt worden ist und bevor der Artikel vulkanisiert worden ist - in den Naturkautschuklatex überall eine wirksame Menge des antimikrobiellen Mittels einzuarbeiten. Zwar werden in dieser Druckschrift auch Katheter genannt, das Dokument fokussiert jedoch ausschließlich auf die Herstellung des entsprechenden Gummiartikels, nicht aber auf eine nachträgliche Desinfektion bzw. auf eine Verhinderung von Inkrustationen des bereits gelegten Katheters.

Zudem betrifft die EP 0 937 394 B1 bzw. das hieraus hervorgegangene deutsche Patent DE 699 21 867 T2 eine antimikrobielle Zusammensetzung, welche einen antimikrobiellen Wirkstoff, der ausgewählt ist aus der Gruppe aus mehr als 30 Vol.-% Alkohol und einer effektiven Menge an Triclosan, und eine effektive Menge von Phenoxyethanol, Benzalkoniumchlorid oder Benzethoniumchlorid sowie schließlich Cocophosphatidyl-Dimoniumchlorid enthält. Diese Druckschrift betrifft also spezielle Kombinationen von Triclosan mit den vorgenannten Inhaltsstoffen. Die dort beschriebene antimikrobielle Zusammensetzung soll topisch auf ein Substrat aufgetragen werden.

Die Publikation gemäß Jones G. LI. et al., "Effect of triclosan on the development of bacterial biofilms by urinary tract pathogens on urinary catheters", Journal of Antimicrobial Chemotherapy, 2006, 57, Seiten 266 bis 272, betrifft die Untersuchung des Einflusses von Triclosan auf die Bildung von Biofilmen durch Harnwegserreger auf der Oberfläche von Kathetern sowie die Untersuchung der Diffusion von Triclosan durch den Rückhalteballon des Harnwegkatheters.

Weiterhin betrifft die Publikation gemäß Jones G. I. et al., "Inhibition of crystalline proteus mirabilis biofilm formation on urinary catheters by Triclosan", Abstracts of General Meeting of the American Society for Microbiology, 2003, 103, Seiten 541 und 542, die Untersuchung der Inhibierung der kristallinen Biofilmbildung bei Zugabe einer Triclosan-Lösung durch den Blasenballon des Katheters in die Blase.

Darüber hinaus betrifft die Publikation gemäß Skaare A. B. et al., "Does the nature of the solvent affect the anti-inflammatory capacity of triclosan?", Joumal of Clinical Periodontology, 1997, 24, Seiten 124 bis 128, die Untersuchung des Einflusses des Lösungsmittels auf die antiinflammatorische Wirkung von Triclosan.

Die Druckschrift US 5 145 667 A betrifft eine lagerstabile Zahnpasta mit Schutz vor Zahnstein, welche neben einem Tripolyphosphatsalz auch eine nichtkationische antibakterielle Substanz wie Triclosan enthalten kann.

Zudem betrifft die Publikation gemäß Kjaerheim V. et al., "Organic solvents and oils as vehicles for triclosan in mouthrinses: a clinical study", Scandinavian Journal of Dental Research, 1994, 102, Seiten 306 bis 308, einen Vergleich der klinischen Wirksamkeit von Triclosan in Mundspüllösungen bei Verwendung unterschiedlicher Lösungsmittel, wie Ölen, Glycerol und Polyethylenglykol.

Weiterhin betrifft die Druckschrift WO 01/76548 A1 ein flüssiges Zahnreinigungsgel mit einem antimikrobiellen Wirkstoff in Form von Triclosan, Hexetidin und Gemischen davon zur Bekämpfung von Zahnbelag.

Darüber hinaus betrifft die Druckschrift US 2002/0125462 A1 eine kosmetische oder topische Zusammensetzung, welche eine Phophatverbindung zur Vermeidung der Korrosion der metallischen Behältnisse, in denen die Zusammensetzung aufbewahrt wird, enthält.

Die Druckschrift US 2002/0051797 A1 betrifft eine Zusammensetzung aus einer hydrophilen und einer hydrophoben Polymeremulsion, welche gegebenenfalls weitere biozide Substanzen mit antimikrobiotischer und antiviraler Wirkung enthalten kann.

Weiterhin betrifft die Druckschrift EP 0 455 475 A2 die Verwendung von Triclosan zur Herstellung von Medikamenten für die Behandlung von Magen-Darm-Erkrankungen, welche mit einer Infektion durch *Heliobacter pylori* einhergehen.

Darüber hinaus betrifft die Druckschrift EP 0 220 890 A2 eine Zusammensetzung zur Mundpflege, welche Triclosan und Polyethylenglykol enthält.

Zudem betrifft die Druckschrift WO 03/006071 A1 ein Verfahren zur Verminderung der Verschleppung von pathogenen Organismen zwischen einem Pfleger und einem Patienten durch Verwendung eines Kits mit zwei Zusammensetzungen, wobei die zweite Zusammensetzung ein Biozid enthält.

Zudem betrifft die Druckschrift US 2005/0215978 A1 eine Vorrichtung zur Infusion einer Blockungslösung in das Lumen eines Katheters, wobei die Vorrichtung eine Spritze und eine in der Spritze befindliche, sterilisierte Blockungslösung mit einem Citratsalz aufweist.

Schließlich betrifft die Druckschrift US 2003/0144362 A1 ein Verfahren zur Blockierung eines Katheters, wobei eine Flüssigkeit in das Lumen eines Katheters eingebracht wird, um das Eindringen bzw. den Fluß von Körperflüssigkeiten in das Katheterlumen bei Verwendung des Katheters zu verringern.

Insgesamt sind somit im Stand der Technik Zusammensetzungen bzw. hiermit modifizierte Vorrichtungen, wie Katheter, beschrieben, welche nicht zu einer effizienten und ausreichenden Vermeidung bzw. Verringerung der Bildung von Inkrustationen insbesondere bei Dauerkathetern führen.

Somit liegt der vorliegenden Erfindung die Aufgabe zugrunde, eine Vorrichtung zur Katheterisierung, insbesondere zur Katheterisierung der Harnblase, enthaltend eine Zusammensetzung, insbesondere eine pharmazeutische Zusammensetzung, bereitzustellen, wobei sich die Zusammensetzung insbesondere zur Verwendung als Blockiermittel bzw. Blockungsmittel für Katheter, insbesondere Harnblasenkatheter, eignet und wobei die Zusammensetzung in effektiver und dauerhafter Weise die Bildung von Inkrustationen, insbesondere bei Dauerkathetern, welche in die Harnblase appliziert sind, vermeiden bzw. verringern soll. Darüber hinaus soll eine Vorrichtung zur Katheterisierung, insbesondere zur Katheterisierung der Harnblase, enthaltend eine Zusammensetzung, bereitgestellt werden, welche die zuvor geschilderten Nachteile des Standes der Technik zumindest weitgehend vermeidet oder aber wenigstens abschwächt. Dabei soll insbesondere eine bessere Prävention bzw. Verhinderung und/oder Verringerung von Inkrustationen in bezug auf in die Harnblase applizierten Kathetern gewährleistet sein.

Zur Lösung des zuvor geschilderten Problems schlägt die vorliegende Erfindung somit eine Vorrichtung zur Katheterisierung, insbesondere zur Katheterisierung der Harnblase, nach Anspruch 1 vor. Weitere, vorteilhafte Eigenschaften der erfindungsgemäßen Vorrichtung sind Gegenstand der diesbezüglichen Vorrichtungsunteransprüche.

Gegenstand der vorliegenden Erfindung - gemäß einem ersten Aspekt - ist somit eine Vorrichtung zur Katheterisierung, insbesondere zur Katheterisierung der Harnblase, enthaltend eine Zusammensetzung, insbesondere pharmazeutische Zusammensetzung, in Form einer flüssigen Zubereitung, wobei sich die Zusammensetzung vorzugsweise zur Verwendung als Blockiermittel (Blockungsmittel) für Katheter, insbesondere Harnblasenkatheter eignet und wobei die Zusammensetzung - in Kombination und jeweils in wirksamen, insbesondere pharmazeutisch wirksamen Mengen und jeweils bezogen auf das Gewicht der Zusammensetzung -
(a) 0,01 bis 5 Gew.-% Triclosan und
(b) 45 bis 95 Gew.% mindestens eines Polyalkylenglykols mit einer mittleren Molmasse (mittlerem Molekulargewicht) von 300 bis 1.000 g/mol
enthält.

Der Begriff "pharmazeutische Zusammensetzung" bzw. "pharmazeutische Zubereitung" oder dergleichen, wie er im Rahmen der vorliegenden Erfindung verwendet wird, ist sehr umfassend zu verstehen und bezeichnet nicht nur pharmazeutische Präparate bzw. Pharmazeutika als solche, sondern auch sogenannte Medizinprodukte oder dergleichen.

Weiterhin bezieht sich der Begriff "Blockiermittel" bzw. "Blockungsmittel" auf eine Zusammensetzung, welche sich insbesondere zur Blockierung bzw. Blockung von Kathetern während ihrer Applikationsdauer eignet. Bei den Kathetern handelt es sich im Rahmen der vorliegenden Erfindung vorzugsweise um Harnblasenkatheter, welche sich insbesondere zu Langzeitkatheterisierung eignen, wobei derartige Katheter vorzugsweise als sogenannte Ballonkatheter insbesondere in der zuvor beschriebenen Weise ausgebildet sind. Eine Blockierung bzw. Blockung - also ein Fixieren bzw. Festsetzen des Katheters - in der Harnblase erfolgt dabei durch ein Aufweiten bzw. Dilatieren der Erweiterungsvorrichtung bzw. des Ballons des Katheters, wozu die erfindungsgemäß vorgesehene Zusammensetzung eingesetzt werden kann. Dies kann im Rahmen der vorliegenden Erfindung durch ein gezieltes Einbringen, vorzugsweise unter leichtem bis mäßigem Druck, der erfindungsgemäß vorgesehenen Zusammensetzung in den Ballon, beispielsweise über ein in den Katheter integriertes Schlauch- bzw. Zuleitungssystem, realisiert werden, wozu insbesondere eine Applikationsvorrichtung eingesetzt werden kann, welche nach der Erfindung die Zubereitung enthält.

Bei allen nachstehend genannten relativen bzw. prozentualen gewichtsbezogenen Mengenangaben ist zu beachten, daß diese im Rahmen der erfindungsgemäß vorgesehenen Zusammensetzung bzw. Kombination vom Fachmann derart auszuwählen sind, daß sie sich unter Einbezug der gegebenenfalls weiteren, erfindungsgemäß vorgesehenen Komponenten, insbesondere Wasser, in der Summe stets zu 100 Gew.-% ergänzen Dies versteht sich für den Fachmann aber von selbst.

Die vorliegende Erfindung zeichnet sich durch den Einsatz von Triclosan und durch die Verwendung eines Polyols zur Bereitstellung einer im Rahmen der erfindungsgemäß vorgesehenen Verwendung hocheffizienten und wirksamen Zusammensetzung aus.

Denn die Anmelderin hat in völlig überraschender Weise herausgefunden, daß durch den Einsatz einer speziellen Substanz mit antimikrobiellen Eigenschaften, nämlich Triclosan in Kombination mit mindestens einem Polyol, eine Zusammensetzung bereitgestellt wird, welche sich insbesondere zur Verwendung als Blockiermittel bzw. Blockungsmittel für Katheter eignet und hervorragende Ergebnisse in bezug auf die Prävention bzw. Verhinderung bzw. Verringerung von Inkrustationen auf bzw. an Harnblasenkathetern liefert. Eine Besonderheit der vorliegenden Erfindung ist darin zu sehen, daß die Zusammensetzung als solche nicht im direkten Kontakt mit dem Körper bzw. dem Körpergewebe steht, sondern vielmehr in einem definierten Lumen bzw. Hohlraum des Katheters vorliegt, beispielsweise in der Erweiterungsvorrichtung bzw. in dem Ballon des Katheters und/oder in dessen Versorgungsschlauch. Nach der Erfindung ist die Zusammensetzung somit durch die Katheterwandung vom Körper bzw. vom umgebenden Gewebe abgetrennt bzw. separiert. Durch diese Separierung wird - ohne sich auf eine spezielle Theorie festlegen zu wollen - eine gezielte bzw. kontrollierte Abgabe der Wirksubstanzen, vorzugsweise von Triclosan - insbesondere durch die Katheterwandung insbesondere durch Permeations- bzw. Diffusionsvorgänge einerseits in Richtung des an den Katheter angrenzenden Gewebes und andererseits in Richtung des Lumens des vorzugsweise harnableitenden Schlauches des Katheters gewährleistet. Dies führt zu einer kontrollierten und gezielten Dosierung, wobei die Wirksubstanz über einen großen Zeitraum in gleichmäßiger Menge bzw. gleichmäßigen Konzentrationen über die Wandung des Katheters abgegeben wird, was die Effizienz insbesondere bei Langzeitkatheterisierungen signifikant erhöht. Gleichzeitig sind die Nebenwirkungen verringert.

Eine gegebenenfalls zusätzliche Behandlung des Katheters, beispielsweise eine aufwendige imprägnierung bzw. Beschichtung des Kathetermaterials vor seiner Anwendung bzw. während seiner Herstellung, entfällt vollstandig. Im Rahmen der Erfindung ist es vielmehr gelungen, eine Zusammensetzung bereitzustellen, welche gewissermaßen eine Doppelfunktion erfüllt: Zum einen eignet sich die Zusammensetzung als solche insbesondere aufgrund ihrer physikochemischen Eigenschaften zur Verwendung als Blockungsmittel für Katheter, d. h. sie kann ohne Schwierigkeiten in den Katheterballon eingebracht werden. Zum anderen führt sie zu einer Verhinderung bzw. Verringerung von Inkrustationen auf dem Katheter.

Das erfingdungsgemäß verwendete Triclosan zeichnet sich dadurch aus, daß es neben einer antibakteriellen Wirkung auch antimykotische und antivirale Eigenschaften aufweist und somit zu einem insgesamt guten Wirkspektrum führt. Insbesondere erweist sich Triclosan als hacheffektiv gegenüber den die Inkrustationen hervorrufenden Bakterienspezies, wie beispielsweise *Proteus mirabilis.* Diesbezüglich kann die Wirksamkeit von Triclosan - ohne sich auf eine Theorie festlegen zu wollen - darauf zurückgeführt werden, daß Triclosan spezifisch die im Rahmen der Fettsäuresynthese der Bakterien notwendige Enoylacylcarrierprotein-Reduktase hemmt, was zu einer Destabilisierung der Bakterienmembran und somit zu einer Zerstörung der Bakterien führt. Darüber hinaus zeichnet sich Triclosan durch seine gute Verträglichkeit aus. Diesbezügliche Studien zeigten weder eine karzinogene noch eine mutagene oder teratogene Wirkung. Triclosan ist der internationale Freiname für 5-Chlor-2-(2,4-dichlorphenoxy)-phenol bzw. 2,2,4-Trichlor-2-2-hydroxydi-phenylether.

Für weitergehende Einzelheiten zu Triclosan kann auf Römpp Chemielexikon, 10. Auflage, Band 6, 1999, Georg Thieme Verlag Stuttgart/New York, Seite 4647, Stichwort: "Triclosan", sowie die dort referierte Literatur verwiesen werden, wobei der gesamte Offenbarungsgehalt der vorgenannten Literatur hiermit durch Bezugnahme eingeschlossen ist.

Die vorliegende Erfindung zeichnet sich durch die Verwendung von bestimmten Mengen an Triclosan aus. So ist das Triclosan in relativen, gewichtsbezogenen Mengen von 0,01 bis 5 Gew.-%. bevorzugt 0,05 bis 3 Gew.-%. vorzugsweise 0,1 bis 2 Gew.-%, bevorzugt 0,1 bis 1 Gew-%, besonders bevorzugt 0,15 bis 0,5 Gew.-%, ganz besonders bevorzugt 0,2 bis 0,4 Gew.-%, in der Zusammensetzung vorhanden, bezogen auf das Gewicht der Zusammensetzung. Erfindungsgemäß ist es gelungen, bereits bei sehr geringen Konzentrationen bzw. Mengen an Triclosan als Wirksubstanz zu einer hohen Wirksamkeit der Zusammensetzung zu gelangen, was zu verringerten Herstellungskosten bei gleichzeitig guter Verträglichkeit führt. Dennoch kann es einzelfallbedingt oder anwendungsbezogen erforderlich sein, von der vorgenannten Mengen abzuweichen, ohne daß der Rahmen der vorliegenden Erfindung verlassen ist. Somit kann die Menge an Triclosan in weiten Bereich variieren.

Der volumenbezogene Wirkstoffgehalt an Triclosan kann 0,1 bis 50 mg/ml, insbesondere 0,5 bis 25 mg/ml, bevorzugt 1 bis 10 mg/ml, besonders bevorzugt 2 bis 5 mg/ml, betragen, bezogen auf das Volumen der Zusammensetzung. Gemäß einer besonders bevorzugten Ausführungsform kann die Zusammensetzung etwa 3,25 mg/ml Triclosan enthalten.

Was das erfindungsgemäß eingesetzte mindestens eine Polyol anbelangt, so handelt es sich hierbei um ein Polyalkylenglykol. Erfindungsgemäß besonders bevorzugt ist die Verwendung eines Polyethylenglykols.

Das erfindungsgemäß verwendete Polyol, welches ein Polyalkylenglykol ist und vorzugsweise ein Polyethylenglykol sein kann, zeichnet sich dadurch aus, daß es eine mittlere Molmasse - im Rahmen der vorliegenden Erfindung synonym auch als Molekulargewicht bezeichnet - von 300 bis 1.000 g/mol, insbesondere 325 bis 750 g/mol, bevorzugt 350 bis 600 g/mol, besonders bevorzugt 375 bis 450 g/mol, ganz besonderes bevorzugt etwa 400 g/mol, aufweist. So kann erfindungsgemäß in bevorzugter Weise beispielsweise Macrogol 400 eingesetzt werden. Hierbei handelt es sich um ein Polyethylenglykol mit einem mittleren Molekulargewicht von 400 g/mol.

Denn die Anmelderin hat nicht zuletzt aufgrund ihrer intensiven Forschungstätigkeit in überraschender Weise herausgefunden, daß die Verwendung eines bestimmten Polyols der vorgenannten Art zu einer hinsichtlich der Prävention bzw. Verringerung von Inkrustationen besonders geeigneten Zusammensetzung im Rahmen eines Blockierungsmittels bzw. Blockungsmittels für Katheter führt. Denn - ohne sich auf diese Theorie festlegen zu wollen - führt die molekulare Struktur des Polyols, insbesondere des Polyethylenglykols der vorgenannten Art, sozusagen zur Ausbildung einer molekularen Matrix in der Zusammensetzung, in welche das Triclosan eingebettet bzw. eingelagert ist, wodurch die Abgabe von Triclosan an die Umgebung, einhergehend mit der Permeation bzw. Diffusion der Wirksubstanz durch die Wandung des Katheters, weiter kontrolliert bzw. verzögert bzw. retardiert ist. Dies führt zu einer gleichmäßigen Langzeitdosierung einhergehend mit einem außerordentlich guten Langzeiteffekt.

Weiterhin kann mit den vorgenannten Polyolen, insbesondere Polyethylenglykol, die Viskosität der Zusammensetzung gezielt eingestellt werden, was insbesondere auch im Hinblick auf die Verwendung als Blockiermittel von hoher Bedeutung ist, da dies die Applizierbarkeit der Zusammensetzung in den Katheter maßgeblich beeinflussen kann. Ein weiterer Vorteil der Verwendung eines Polyols, insbesondere eines Polyethylenglykols der vorgenannten Art, ist darin zu sehen, daß die Zusammensetzung insgesamt über eine deutlich verbesserte Materialverträglichkeit verfügt, da die Substanzen das Kathetermaterial nicht angreifen. Weiterhin weist Polyethylenglykol beispielsweise im Vergleich zu niederen Alkoholen beim Patienten eine deutlich bessere Verträglichkeit auf.

Was das mindestens eine Polyol, insbesondere Polyethylenglykol, weiterhin anbelangt, so liegt dies in bezug auf die erfindungsgemäß vorgesehene Zusammensetzung in Mengen von 45 bis 95 Gew.-%, insbesondere 50 bis 90 Gew.-%, vorzugsweise 55 bis 85 Gew.-%, bevorzugt 60 bis 85 Gew.-%, besonders bevorzugt 65 bis 80 Gew.-%. ganz besondere bevorzugt 70 bis 80 Gew.-%. bezogen auf das Gewicht der Zusammensetzung, vor. Weiterhin liegt das (b) eine Polyol, insbesondere Polyethylenglykol, in Mengen von mindestens 45 Gew.-%, insbesondere in Mengen von mindestens 50 Gew.-%. bezogen auf das Gewicht der Zusammensetzung, vor. Denn die Anmelderin hat völlig überraschend herausgefunden, daß insbesondere bei Gehalten von mindestens 45 Gew.-% oder mehr eine besonders gute Wirkung der erfindungsgemäß vorgesehenen Zusammensetzung vorliegt, was - ohne sich hierauf festlegen zu wollen - durch eine optimierte Ausbildung der zuvor beschriebenen Matrix erklärt werden kann.

Bezug nehmend auf den volumenbezogenen Gehalt an dem mindestens einen Polyol, vorzugsweise Polyethylenglykol, so kann dieses im Bereich von 495 bis 1.050 mg/ml, insbesondere 550 bis 1.000 mg/ml, vorzugsweise 600 bis 950 mg/ml, bevorzugt 590 bis 660 mg/ml, besonders bevorzugt 715 bis 900 mg/ml, ganz besonders bevorzugt 770 bis 900 mg/ml, in der Zusammensetzung vorliegen, bezogen auf das Volumen der Zusammensetzung.

Im Rahmen der vorliegenden Erfindung ist es darüber hinaus gelungen, die Wirksamkeit der erfindungsgemäß vorgesehenen Zusammensetzung in bezug auf die Prävention bzw. Verringerung von Inkrustationen an der zum Gewebe orientierten Katheterwand bzw. an der zum Lumen, insbesondere des Schlauches zur Harnabführung, orientierten Katheterwandung durch eine gezielte Abstimmung des Mengenverhältnisses zwischen Triclosan einerseits und dem mindestens einen Polyol andererseits zu erhöhen. So kann es erfindungsgemäß vorgesehen sein, daß das Mengenverhältnis von Triclosan und dem mindestens einem Polyol, vorzugsweise Polyethylenglykol, in der Zusammensetzung im Bereich von 1 : 10.000 bis 1 : 10, insbesondere 1 : 2.000 bis 1 : 20, vorzugsweise 1 : 1.000 bis 1 : 50, bevorzugt 1 : 500 bis 1 : 100, besonders bevorzugt 1 : 300 bis 1 : 200, liegt.

Denn der Anmelderin ist es gelungen, durch die gezielte Anpassung und Abstimmung der jeweiligen Wirksubstanzen der binären erfindungsgemäß vorgesehenen Zusammensetzung zu einer besonders guten Wirksamkeit hinsichtlich der Prävention bzw. Verringerung von Inkrustationen zu gelangen, da - ohne sich auf eine Theorie festlegen zu wollen - insbesondere bei den vorgenannten Mengenverhältnissen eine gute Interaktion zwischen Triclosan einerseits und dem mindestens einem Polyol andererseits vorliegt, was die gezielte Abgabe der Wirksubstanz an die Umgebung positiv beeinflußt und zwar dahingehend, daß sich insbesondere auf der Katheterwandung ansiedelnde Bakterien, wie *Proteus mirabilis,* in effektiver Weise vermindert bzw. abgetötet werden. Als Folge wird die Bildung von Inkrustationen deutlich vermindert bzw. diese treten gar nicht erst auf. Der Leidensdruck der Patienten ist deutlich verringert, und die Katheter können für einen längeren Zeitraum in der Harnblase im Rahmen einer Langzeitkatheterisierung verbleiben.

Neben den vorgenannten Wirk- und/oder Inhaltsstoffen können außerdem übliche Zusatz- und/oder Hilfsstoffe, insbesondere übliche pharmazeutische Zusatz- und/oder Hilfsstoffe, zugegeben sein. Diese können insbesondere ausgewählt sein aus der Gruppe von Stabilisierungs-, Färbe-, Puffer-, Riech-, Streck-, Binde-, Netz- und/oder Konservierungsstoffen sowie deren Kombinationen. Der Fachmann ist jederzeit in der Lage, die diesbezüglich zu wählenden Mengen in bezug auf die erfindungsgemäß vorgesehene Zusammensetzung anzupassen.

Die erfindungsgemäß vorgesehene Zusammensetzung liegt vorzugsweise in Form einer Lösung oder Suspension vor. Gemäß einer weiteren erfindungsgemäßen Ausführungsform liegt nach der Erfindung die Zusammensetzung insbesondere in Form einer wäßrigen Lösung oder einer wäßrigen Dispersion vor.

In diesem Zusammenhang kann es erfindungsgemäß vorgesehen sein, daß die Zusammensetzung außerdem Wasser enthält. Nach der Erfindung liegt die Zusammensetzung in flüssiger Form vor, so daß sich die erfindungsgemäß vorgesehene Zusammensetzung insbesondere als Blockiermittel bzw. Blockungsmittel für Katheter eignet, da die erfindungsgemäß vorgesehene Zusammensetzung in einfacher Weise appliziert bzw. in den Katheter eingebracht werden kann. Dabei sollte die erfindungsgemäß vorgesehene Zusammensetzung steril sein.

Die erfindungsgemäß vorgesehene Zusammensetzung kann einen pH-Wert von 6 bis 8, insbesondere 6,5 bis 7,8, vorzugsweise 7 bis 7,5, aufweisen.

In erfindungsgemäß bevorzugter Weise beträgt die Dichte der erfindungsgemäß vorgesehenen Zusammensetzung bei einer Temperatur von 20 °C 1 bis 1,4 g/ml), insbesondere 1,025 bis 1,35 g/ml), vorzugsweise 1,05 bis 1,3 g/ml, bevorzugt 1,075 bis 1,25 g/ml, ganz besonders bevorzugt 1,1 bis 1,225 g/ml.

Weiterhin kann die erfindungsgemäß vorgesehene Zusammensetzung bei einer Temperatur von 20 °C eine dynamische Viskosität von 5 bis 1.000 mPas, insbesondere 5 bis 500 mPas, vorzugsweise 7,5 bis 100 mPas, bevorzugt 7,5 bis 50 mPas, besondere bevorzugt 10 bis 20 mPas, aufweisen.

Weiterhin kann die erfindungsgemäß vorgesehene Zusammensetzung bei einer Temperatur von 20 °C einen Brechungsindex von 1,2 bis 1,7, insbesondere 1,25 bis 1,6, bevorzugt 1,3 bis 1,5, aufweisen.

Aufgrund der vorgenannten Eigenschaften, insbesondere hinsichtlich des pH-Wertes, der Dichte sowie der dynamischen Viskosität, eignet sich die erfindungsgemäß vorgesehene Zusammensetzung in besonderer Weise zur Verwendung als Blockierungsmittel für Katheter. Die im Rahmen der vorliegenden Erfindung vorgesehene Viskosität führt gleichermaßen dazu, daß die erfindungsgemäß vorgesehene Zusammensetzung in einfacher Weise in den Katheter eingebracht werden kann.

Gemäß einer erfindungsgemäß besonders bevorzugten Ausführungsform ist nach der vorliegenden Erfindung die Zusammensetzung, welche in Form einer flüssigen Zubereitung vorliegt, durch die folgende Rezeptur gekennzeichnet, wobei alle nachstehend genannten Mengenangaben jeweils auf die erfindungsgemäß vorgesehene Zusammensetzung bezogen sind, wobei die Rezeptur in Kombination enthält:
(a) Triclosan in Mengen von 0,01 bis 5 Gew.-%, bevorzugt 0,05 bis 3 Gew.-%, vorzugsweise 0,1 bis 2 Gew.-%, bevorzugt 0,1 bis 1 Gew.-%, besonders bevorzugt 0,15 bis 0,5 Gew.-%, ganz besonders bevorzugt 0,2 bis 0,4 Gew.-%, bezogen auf das Gewicht der Zusammensetzung, und
(b) mindestens ein Polyol, wobei das mindestens eine Polyol ein Polyalkylenglykol, vorzugsweise ein Polyethylenglykol mit einer mittleren Molmasse (mittlerem Molekulargewicht) von 300 bis 1.000 g/mol, insbesondere 325 bis 750 g/mol, bevorzugt 350 bis 600 g/mol, besonders bevorzugt 375 bis 450 g/mol, ganz besonders bevorzugt etwa 400 g/mol, ist, wobei das mindestens eine Polyol in Mengen von 45 bis 95 Gew.-%, insbesondere 50 bis 90 Gew.-%, vorzugsweise 55 bis 85 Gew.-%, bevorzugt 60 bis 85 Gew.-%, besonders bevorzugt 65 bis 80 Gew.-%, ganz besonders bevorzugt 70 bis 80 Gew.-%, vorliegt, bezogen auf das Gewicht der Zusammensetzung,
insbesondere wobei das Mengenverhältnis von (a) Triclosan einerseits zu (b) dem mindestens einen Polyol, vorzugsweise Polyethylenglykol, andererseits in der Zusammensetzung im Bereich von 1 : 10.000 bis 1 : 10, insbesondere 1 : 2.000 bis 1 : 20, vorzugsweise 1 : 1.000 bis 1 : 50, bevorzugt 1 : 500 bis 1 : 100, besonders bevorzugt 1 : 300 bis 1 : 200, liegt.

Die erfindungsgemäß vorgesehene Zusammensetzung eignet sich insbesondere zur Verwendung als Blockiermittel im Sinne der vorgenannten Definition für Katheter, insbesondere Harnblasenkatheter, wobei es sich diesbezüglich vorzugsweise um Dauerkatheter der vorgenannten Art handelt, welche vorzugsweise im Rahmen einer Langzeitkatheterisierung appliziert werden.

Die Dauerkatheter können insbesondere in Form von Ballonkathetern vorliegen. Die erfindungsgemäß vorgesehene Zusammensetzung eignet sich somit zum Auffüllen von Blockierungseinrichtungen, insbesondere in Form von Blockierungserweiterungen, vorzugsweise Blockierungsballonen, von Kathetern, insbesondere Harnblasenkathetern, vorzugsweise Dauerkathetem der vorgenannten Art. Dabei kann die Zusammensetzung beispielsweise mit Hilfe einer Spritze oder dergleichen über ein Schlauchsystem in die Blockierungseinrichtung des Katheters appliziert werden, was zu einer Dilatation bzw. Erweiterung derselbigen und somit zu einem Blockieren bzw. zu einer Blockung des Katheters nach erfolgter Applikation in die Harnblase führt.

Die erfindungsgemäß vorgesehene Zusammensetzung eignet sich zur Verhinderung und/oder Verringerung von insbesondere während der Applikationsdauer hervorgerufenen bzw. auftretenden Inkrustationen an Kathetern, insbesondere Harnblasenkathetern, vorzugsweise Dauerkathetern, insbesondere wobei es sich bei der Inkrustation um eine bakteriell hervorgerufene Inkrustation handelt. Hierbei kommen insbesondere die zuvor genannten Bakterien bzw. Bakterienstämme in Betracht, welche ein den im Urin vorhandenen Harnstoff spaltendes Enzym, insbesondere Urease, aufweisen. Insbesondere handelt es sich bei dem Bakterium um *Proteus mirabilis.*

Was nach der Erfindung die Zusammensetzung weiterhin anbelangt, so ist diese vorzugsweise dosierfertig bzw. applikationsfertig in ein Behältnis eingebracht. Dabei können die Behältnisse beispielsweise in Form von sterilen Spritzen oder dergleichen vorliegen. Die Volumengröße der Zusammensetzung kann in den Behältnissen 1 bis 50 ml, insbesondere 2 bis 30 ml, bevorzugt 5 bis 15 ml, besonders bevorzugt etwa 10 ml, betragen, je Behältnis bzw. Applikationseinheit (Dosiereinheit).

Im Rahmen der vorliegenden Erfindung wird gleichermaßen ein Behältnis, welches die erfindungsgemäß vorgesehene Zusammensetzung enthält, verwendet. Das erfindungsgemäß vorgesehene Behältnis kann insbesondere in Form einer Spritze, vorzugsweise einer sterilen Spritze, vorlieben. Dabei kann das Behältnis eine Anschlußvorrichtung für die der Versorgung der Erweiterungsvorrichtung des Katheters dienenden Versorgungsvorrichtung, beispielsweise in Form eines Schlauches, aufweisen. Das erfindungsgemäß vorgesehene Behältnis sollte vorzugsweise einen sterilen Verschluß aufweisen.

Was die zuvor beschriebene erfindungsgemäße Vorrichtung zur Katheterisierung, insbesondere zur Katheterisierung der Harnblase, welche die erfindungsgemäß vorgesehene Zusammensetzung enthält, weiterhin anbelangt, so kann es diesbezüglich vorgesehen sein, daß die erfindungsgemäße Vorrichtung beispielsweise einen Katheter, vorzugsweise einen Dauerkatheter mit einer Aufnahmevorrichtung für die erfindungsgemäß vorgesehene Zusammensetzung, beispielsweise in Form einer Blockierungseinrichtung, vorzugsweise eines Blockierungsballons, und/oder mit einer Versorgungsvorrichtung für den Blockierungsballon, beispielsweise in Form eines Schlauches, aufweist. Über diesen Zuführschlauch kann das Befüllen oder Entleeren des Blockierungsballons mit der erfindungsgemäß vorgesehenen Zusammensetzung durchgeführt werden.

Bei dem im Rahmen der erfindungsgemäßen Vorrichtung eingesetzten Katheter kann es sich somit um einen für die Langzeitkatheterisierung vorgesehenen Dauerkatheter handeln, welcher beispielsweise als transurethaler oder suprapubischer Blasenkatheter ausgebildet sein kann. Das zur Herstellung des Katheters eingesetzte Material kann dabei insbesondere eine flexible Zusammensetzung, wie Latex, Polyvinylchlorid und/oder Silikon, sein. Zudem kann es vorgesehen sein, daß der im Rahmen der erfindungsgemäßen Vorrichtung eingesetzte Katheter zudem eine hydrophile Beschichtung aufweist, welche die Gleitfähigkeit zusätzlich erhöht. Diesbezüglich kann auch eine dünne Beschichtung aus einem diamantähnlichen Kohlenstoff ("*diamond-likecarbon*") vorgesehen sein, was zu einer weiteren Reduzierung der Keimbesiedlung auf dem Katheter führen kann. Bei der Auswahl des Materials ist zu beachten, daß die Diffusion der Wirksubstanz der erfindungsgemäß vorgesehenen Zusammensetzung, insbesondere von Triclosan, durch die Katheterwandung nicht negativ beeinträchtigt ist.

Was den Katheter, insbesondere Harnblasenkatheter anbelangt, so kann dieser im Falle einer zuvor beschriebenen Zweiwegekonstruktion beispielsweise derart konzipiert sein, daß der insbesondere zur Harnableitung dienende Schlauch von dem zur Versorgung der Ballonvorrichtung dienenden Schlauch geführt bzw. umhüllt ist. Mit anderen Worten kann der zur Harnableitung dienende Schlauch gewissermaßen vollumfänglich von dem zur Versorgung der Erweiterungsvorrichtung dienenden Schlauch, welcher die erfindungsgemäß vorgesehene Zusammensetzung beinhaltet bzw. führt, umgeben sein. Hierdurch wird eine ganzflächige Diffusion der Wirksubstanzen auch durch die Wandung des zur Harnableitung dienenden Schlauches gewährleistet.

Im Rahmen der vorliegenden Erfindung wird also die zuvor beschriebene erfindungsgemäß vorgesehene Zusammensetzung als Blockiermittel für Katheter, insbesondere Harnblasenkatheter, vorzugsweise Dauerkatheter, insbesondere in Form von Ballonkathetern bzw. zum Befüllen von Blockierungseinrichtungen von Kathetern, insbesondere in Form von Blockierungserweiterungen, vorzugsweise Blockierungsballonen, verwendet.

Dabei ist es im Rahmen der vorliegenden Erfindung gelungen, die Zusammensetzung derart abzustimmen, daß eine gezielte, kontrollierte und langfristig sichergestellte Abgabe der Wirksubstanz durch die Wandung des Katheters in die Umgebung ermöglicht wird, so daß die Wirksubstanz ihre antimikrobielle Wirkung in bezug auf Inkrustationen verursachende Erreger bzw. Bakterien entfalten kann.

Was die efindungsgemäß vorgesehene Verwendung der zuvor beschriebenen Zusammensetzung weiterhin anbelangt, so kann das Befüllen der Blockierungseinrichtung bzw. des Ballons des Katheters nach Setzen bzw. Einführen des Katheters in die Harnblase durchgeführt werden. Zu Zwecken des Be- bzw. Auffüllens der Blockierungseinrichtung kann beispielsweise eine vorzugsweise sterile Spitze der vorgenannten Art verwendet werden.

Die bezüglich des Auffüllens des Ballons verwendete Menge bzw. das diesbezügliche Volumen der zuvor beschriebenen erfingdungsgemäß vorgesehenen Zusammensetzung kann dabei in Abhängigkeit von dem jeweiligen Katheter variiert werden bzw. auf diesen abgestimmt werden. Im allgemeinen beträgt das in den Katheterballon applizierte Volumen etwa 1 bis 50 ml.

Schließlich werden im Rahmen der vorliegenden Erfindung die zuvor beschriebene erfindungsgemäß vorgesehene Zusammensetzung bzw. die zuvor beschriebene erfindungsgemäße Vorrichtung zur Verhinderung bzw. Verringerung von insbesondere während der Applikationsdauer des Katheters hervorgerufenen Inkrustationen am Katheter, insbesondere an einem Harnblasenkatheter, wobei es sich diesbezüglich - wie zuvor geschildert - vorzugsweise um einen Dauerkatheter handelt, verwendet.

Die mit der zuvor beschriebenen erfindungsgemäß vorgesehenen Zusammensetzung ausgestatteten Kathetersysteme können über eine im Vergleich zum Stand der Technik signifikant verlängerte Applikationsdauer eingesetzt werden, so daß ein häufiger Wechsel des Katheters vermieden wird. Dies führt zusätzlich zu einer deutlichen Kostenreduktion bei gleichzeitig verbessertem Befinden des Patienten, insbesondere da aufgrund der Vermeidung bzw. Verringerung von Inkrustationen beispielsweise beim Entfernen bzw. Ziehen des Katheters deutlich weniger Schmerzen auftreten und das Gewebe im Kontaktbereich des Katheters nicht beschädigt wird.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne daß er dabei den Rahmen der Erfindung verläßt.

### Ausführungsbeispiele:

Im Rahmen von seitens der Anmelderin durchgeführten Ausführungsbeispielen bzw. Wirksamkeitsstudien wurde die herausragende Wirkung einer zuvor beschriebenen erfindungsgemäß vorgesehenen Zusammensetzung auf Basis der erfindungsgemäß vorgesehenen Wirkstoffkombination unter Verwendung eines Blasenmodells sowie im Rahmen eines Einsatzes am Patienten belegt.
1. Im Rahmen einer ersten Versuchsreihe wurden verschiedene Zusammensetzungen an einem Blasenmodell getestet. Diesbezüglich wurden Ballonkatheter aus weichem Silikon (Modell Ch. 14 Uro 1.100-14, MTR, Medizintechnik GmbH, Reutlingen) sowie als Blasenersatz Infusionsbeutel mit einem Volumen von 3.000 ml (IV-Container, iphas Pharma-Verpackung, Würselen) verwendet.
Zur Versuchsdurchführung wurden die Katheter in eine kleine Zuführung der IV-Container eingeklebt. Die Ballons wurden anschließend mit je 10 ml der nachfolgend beschriebenen Zusammensetzung gefüllt. Über eine zweite Zuführung wurde künstlicher Urin in die IV-Container eingefüllt, bis das "Auge" der Katheter - also die in das Blasenmodell eingeführte Öffnung des Katheters - vom Flüssigkeitsspiegel erreicht wurde. Anschließend wurde die Lösung im IV-Container mit *Proteus mirabilis* angeimpft. Über eine Peristaltikpumpe wurde künstlicher Urin mit einer Flußrate von etwa 0,5 ml pro Minute kontinuierlich bis zum Ende der Laufzeit von 72 h zugeführt. Im Abstand von ca. 24 h wurde die Keimzahl im IV-Container, der pH-Wert im IV-Container und die Katheter in bezug auf Verstopfungen kontrolliert und dokumentiert.
(a) Im Rahmen von zwei Placebountersuchungen, bei welcher eine Blockerzusammensetzung ohne Verwendung von Triclosan und ohne Polyol eingesetzt wurde, trübte sich der künstliche Urin in den IV-Containern nach 12 h ein, der pH-Wert stieg bis 24 h nach dem Start von 5,8 auf 9 an. Die Keimzahl war bis zur ersten Bestimmung nach 24 h bereits auf über 100 Millionen keimbildende Einheiten pro Milliliter (KBE/ml) angewachsen. Nach 30 bis 40 h waren die Katheter der Placebogruppen durch Ablagerungen blockiert, der Versuch wurde an dieser Stelle abgebrochen.
(b) Im Rahmen einer zweiten Versuchsreihe wurden Blockerzusammensetzungen mit verschiedenen Triclosanmengen (0,3 Gew.-%, 1 Gew.-%, 5 Gew.-%) und mit einem relativ niedrigen Polyethylenglykolgehalt von 5 Gew.-% eingesetzt, wobei das verwendete Polyethylenglykol (Macrogol 400) eine mittlere Molmasse von etwa 400 g/mol aufwies. Im einzelnen:
Zusammensetzung A:

| | |
|---|---|
| Triclosan | 0,3 Gew.-% |
| Polyethylenglykol, M = 400 g/mol | 5 Gew.-% |
| gereinigtes Wasser | 94,7 Gew.% |

Zusammensetzung B:

| | |
|---|---|
| Triclosan | 1 Gew.-% |
| Polyethylenglykol, M = 400 g/mol | 5 Gew.-% |
| gereinigtes Wasser | 94 Gew.-% |

Zusammensetzung C:

| | |
|---|---|
| Triclosan | 5 Gew.-% |
| Polyethylenglykol, M = 400 g/mol | 5 Gew.-% |
| gereinigtes Wasser | 90 Gew.-% |

Der pH-Wert stieg in dem Versuch mit der Zusammensetzung A bis 24 h nach dem Start von 5,8 auf 7,2 und nach 72 h auf 7,8; die Keimzahl war bis zur ersten Bestimmung nach 24 h auf 10 Millionen KBE/mli und nach 72 h auf 18 Millionen KBE/ml angestiegen. Nach etwa 50 h waren an den Kathetern geringe Inkrustationen zu beobachten.
In bezug auf die Zusammensetzung B wurde ein Anstieg des pH-Werts bis 24 h nach dem Start von 5,8 auf 7,6 und nach 72 h auf 7,8 beobachtet. Die Keimzahl wuchs nach der ersten Bestimmung nach 24 h auf 9,5 Millionen KBE/ml an und erreichte nach 72 h einen Wert von 16 Millionen KBE/ml. Die Katheter zeigten geringfügige Inkrustationen, wobei der harnableitende Schlauch jedoch nicht verstopft war.
Schließlich wurde in bezug auf die Zusammensetzung C ein Anstieg des pH-Wertes von 5,8 auf 7,5 nach 24 h und auf 7,8 nach 72 h beobachtet. Die Keimzahl stieg von 9,5 Millionen KBE/ml nach 24 h auf 15,5 Millionen KBE nach 72 h.
Die Versuche zeigen, daß bereits bei einer Konzentration von 0,3 Gew.% Triclosan ein signifikanter Effekt zu beobachten ist. Aufgrund des geringen Polyethylengehaltes kam es zu einer leichten Bakterienvermehrung einhergehend mit einer geringfügigen Inkrustation.
(c) Weiterhin wurden Zusammensetzungen in der zuvor beschriebenen Weise untersucht, welche bei einem konstanten Triclosangehalt von 0,3 Gew.-% variable Polyethylenglykolgehalte aufwiesen. Als Polyethylenglykol wurde auch hier ein Polyethylenglykol mit einer Molmasse von 400 g/mol (Macrogol 400) eingesetzt. Im einzelnen:
Zusammensetzung D:

| | |
|---|---|
| Triclosan | 0,3 Gew.-% |
| Polyethylenglykol, M = 400 g/mol | 5 Gew.-% |
| gereinigtes Wasser | 94,7 Gew.-% |

Zusammensetzung E:

| | |
|---|---|
| Triclosan | 0,3 Gew.-% |
| Polyethylenglykol, M = 400 g/mol | 25 Gew.-% |
| gereinigtes Wasser | 74,7 Gew.-% |

Zusammensetzung F:

| | |
|---|---|
| Triclosan | 0,3 Gew.-% |
| Polyethylenglykol, M = 400 g/mol | 45 Gew.-% |
| gereinigtes Wasser | 54,7 Gew.-% |

Zusammensetzung D, welche der Zusammensetzung A im vorgenannten Versuchsbeispiel b) entsprach, führte zu vergleichbaren Ergebnissen: Es wurde ein Anstieg des pH-Wertes von 5,8 auf 7,1 nach 24 h und auf 7,7 nach 72 h beobachtet. Die Keimzahl stieg von 10,5 Millionen KBE/ml nach 24 h auf 17,5 Millionen KBE/ml nach 72 h. Es wurden geringe Inkrustationserscheinungen beobachtet.
In bezug auf die Zusammensetzung E ergab sich ein etwas geringerer Anstieg sowohl in bezug auf den pH-Wert als auch hinsichtlich der Anzahl der koloniebildenden Einheiten im Vergleich zu der Zusammensetzung D: So stieg der pH-Wert 24 h nach Versuchsbeginn von 5,8 auf 6,2 und nach 72 h auf 6,9 an. Die Anzahl der koloniebildenden Einheiten stieg von 10 Millionen KBE/ml nach 24 h auf 12 Millionen KBE/ml nach 72 h an. Inkrustationen konnten nur im geringen Umfang beobachtet werden.
In bezug auf die Zusammensetzung F konnten nochmals verbesserte Werte ermittelt werden: Es resultierte ein Anstieg des pH-Werts von 5,8 auf 6,2 nach 24 h und auf 6,5 nach 72 h. Die Keimzahl stieg von 3 Millionen KBE/ml nach 24 h auf 4 Millionen KBE nach 72 h. Inkrustationen waren praktisch nicht zu beobachten.
Die Versuchsreihe c) zeigt einen signifikanten Einfluß der Polyethylenglykolgehalte auf die Wirksamkeit der Zusammensetzung. Mit zunehmendem Polyethylenglykolgehalt ist ein stärkerer antimikrobieller Effekt der Zusammensetzung zu beobachten, wobei insbesondere auch die Langzeitwirkung verbessert ist.
Dies kann, ohne sich auf diese Theorie festlegen zu wollen und wie bereits ausgeführt, damit begründet werden, daß das Triclosan gewissermaßen in eine durch das Polyethylenglykol ausgebildete Matrix eingelagert wird und somit eine kontrollierte bzw. retardierte Freisetzung von Triclosan stattfinden kann, was insbesondere zu einer Verbesserung der Langzeitwirkung führt.
(d) Im Rahmen einer weiteren Versuchsserie wurden Zusammensetzungen untersucht, bei denen die Summe der Gewichtsanteile von Triclosan und Polyethylenglykol (Macrogol 400) 75 Gew.-% betrugen. Auch in dieser Versuchsreihe wurden die Mengen an Triclosan im Bereich von 0,3 Gew.-% bis 5 Gew.-% variiert. Zu den Zusammensetzungen im einzelnen:
Zusammensetzung G:

| | |
|---|---|
| Triclosan | 0,3 Gew.-% |
| Polyethylenglykol, M = 400 g/mol | 74,7 Gew.-% |
| gereinigtes Wasser | 25 Gew.-% |

Zusammensetzung H:

| | |
|---|---|
| Triclosan | 1 Gew.-% |
| Polyethylenglykol, M = 400 g/mol | 74 Gew.-% |
| gereinigtes Wasser | 25 Gew.-% |

Zusammensetzung I:

| | |
|---|---|
| Triclosan | 5 Gew.-% |
| Polyethylenglykol, M = 400 g/mol | 70 Gew.-% |
| gereinigtes Wasser | 25 Gew.-% |

Auch der pH-Wert stieg bei den verschiedenen Triclosanansätzen der Zusammensetzung G bis I nur langsam an und erreichte nach 24 h einen pH-Wert von 6,0 (ausgehend von 5,8). Zum Ende der Laufzeit nach 72 h ergab sich für sämtliche Zusammensetzungen G bis I ein pH-Wert von etwa 6,2. Was die Keimzahl betrifft, so wuchs diese in bezug auf Zusammensetzung G auf maximal 0,3 Millionen KBE/ml nach 72 h, während sie in bezug auf Zusammensetzung H auf 0,5 Millionen KBE/ml nach 72 h und in bezug auf die Zusammensetzung I auf 0,6 Millionen KBE/ml nach 72 h wuchs. Inkrustationen waren nicht zu beobachten. Die Katheter blieben bis zur Beendigung der Versuchsdauer vollständig frei von Inkrustationen.
(e) Schließlich wurde eine Versuchsreihe unter Verwendung von Triclosan und kurzkettigen Alkoholen, wie Ethanol und Propanol (nicht erfindungsgemäß) durchgeführt. Im einzelnen:
Zusammensetzung J (Vergleich):

| | |
|---|---|
| Triclosan | 0,3 Gew.-% |
| Ethanol | 74,7 Gew.-% |
| gereinigtes Wasser | 25 Gew.-% |

Zusammensetzung K (Vergleich):

| | |
|---|---|
| Triclosan | 0,3 Gew.-% |
| Propanol | 74,7 Gew.-% |
| gereinigtes Wasser | 25 Gew.-% |

In diesen Vergleichstests wurde in bezug auf beide Zusammensetzungen eine Erhöhung des pH-Wertes von 5,8 auf 6,8 nach 24 h und auf 7,5 nach 72 h beobachtet werden. Was die Keimzahl anbelangt, so konnte für die Zusammensetzung J ein Wert von 9, Millionen KBE/ml nach 24 h und 9,5 Millionen KBE/ml nach 72 h beobachtet werden. In bezug auf die Zusammensetzung K betrug die Keimzahl nach 24 h 8 Millionen KBE/ml und nach 72 h 9,5 Millionen KBE/ml.
Insgesamt zeigen die Ergebnisse eine deutliche Wachstumshemmung in bezug auf *Proteus mirabilis* für die Triclosanlösungen an. Dabei ist insbesondere zu beobachten, daß der keimhemmende Effekt, welcher mit einer geringeren Veränderung des pH-Wertes sowie einer geringeren Inkrustationsbildung einhergeht, nur geringfügig von der gewählten Triclosanmenge abhängt. So liegt das Wirkoptimum für Triclosan bereits bei sehr kleinen Gehalten bzw. Konzentrationen vor. Denn bereits bei einer Triclosanmenge von 0,3 Gew.-% ist ein signifikanter Effekt zu beobachten, wie es anhand der Zusammensetzungen A und D zu sehen ist, der durch höhere Gehalte kaum noch gesteigert werden kann.
Weiterhin kann festgestellt werden, daß der antimikrobielle Effekt und damit die Verhinderung von Inkrustationen deutlich von dem Gehalt bzw. der Konzentration des Polyols - im vorliegenden Fall ein Polyethylenglykol mit einer mittleren Molmasse von 400 g/mol (Macrogol 400) - beeinflußt wird. So ist mit steigendem Polyethylenglykolgehalt eine Steigerung der antimikrobiellen Wirksamkeit der Zusammensetzung zu beobachten. Insbesondere für Konzentrationen von Polyethylenglykol von mindestens 45 Gew.-% ist eine sehr gute antimikrobielle Wirkung zu beobachten. Insgesamt ist somit festzustellen, daß bereits bei geringen Triclosankonzentrationen eine hohe Effektivität vorliegt, welche durch das Vorhandensein von Polyethylenglykol insbesondere in Konzentrationen von mindestens 45 Gew.-% in signifikanter Weise gesteigert werden kann. Weiterhin konnte gezeigt werden, daß die Verwendung eines Polyols gegenüber der Verwendung von kurzkettigen Alkoholen zu einer signifikanten Steigerung der Wirksamkeit führt.
2. In einem weiteren Versuchskomplex wurde der Einfluß der erfindungsgemäß vorgesehenen Zusammensetzung G sowie der Vergleichszusammensetzung J gemäß Versuchskomplex 1.) auf die Haltbarkeit von Kathetern der zuvor beschriebenen Art untersucht. Diesbezüglich wurden Katheter mit den jeweiligen Zusammensetzungen befüllt und über einen Zeitraum von drei Monaten bei Raumtemperatur gelagert. Während die Katheter, welche mit der Zusammensetzung J gefüllt waren, zum Ende des Versuchszeitpunkts teilweise eine gelbliche Verfärbung der Katheterwandung aufwiesen und das Silikonmaterial des Katheters in einigen Bereichen porös wurde, konnten in bezug auf die erfindungsgemäß vorgesehene Zusammensetzungen G keinerlei Veränderungen in bezug auf das Kathetermaterial beobachtet werden.
3. Schließlich wurde die Wirkung der erfindungsgemäß vorgesehenen Zusammensetzung G sowie der Vergleichszusammensetzung J gemäß Versuchskomplex 1.) an Kathetern untersucht, die zuvor Probanden appliziert bzw. gelegt wurden. Die Katheter wurden entweder mit der Zusammensetzung G oder mit der Zusammensetzung J geblockt. Der Katheter wurde nach einer Woche entfernt und begutachtet.
In bezug auf die Vergleichszusammensetzung J waren deutliche Inkrustationen zu beobachten und der harnableitende Schlauch des Katheters wies deutliche Anzeichen einer durch Inkrustationen verursachten Verengung auf, wobei der Katheter jedoch noch nicht vollständig verschlossen war. Zudem klagten die Probanden Ober Schmerzen nicht nur während des Entfernens des Katheters sondern auch während der Applikationsdauer, was insbesondere mit dem vergleichsweise aggressiven kurzkettigen Alkohol zusammenhängen kann. Diesbezüglich zeigte eine durchgeführte Spiegelung des Urogenitaltraktes deutliche Reizungen im Bereich des Harnleiters und des Mündungsbereiches des Harnleiters in die Harnblase.
Demgegenüber zeigten die Katheter, welche die erfindungsgemäß vorgesehene Zusammensetzung G enthielten, auch nach einer Applikationsdauer von einer Woche keine sichtbaren Inkrustationen. Die Probanden empfanden das Ziehen des Katheters als weniger schmerzhaft. Eine durchgeführte Spiegelung des Urogenitaltraktes zeigte keine signifikante Veränderung bzw. Reizung im Bereich des Harnleiters bzw. im Bereich der Harnleitermündung in die Blase.
Auch dieser Test belegt somit die besonders gute Wirkung der erfindungsgemäß vorgesehenen Kombination gemäß Zusammensetzung G im Vergleich zur Vergleichszusammensetzung J.

Insgesamt zeigen die durchgeführten Versuchsreihen die hervorragende, synergistische Wirkung der erfindungsgemäß vorgesehenen binären Kombination gegenüber den jeweiligen Kontrollexperimenten.

## Patentansprüche

1. Vorrichtung zur Katheterisierung, insbesondere zur Katheterisierung der Harnblase, enthaltend eine Zusammensetzung, insbesondere pharmazeutische Zusammensetzung, in Form einer flüssigen Zubereitung, wobei die Zusammensetzung in Kombination und jeweils in wirksamen, insbesondere pharmazeutisch wirksamen Mengen und jeweils bezogen auf das Gewicht der Zusammensetzung
(a) 0,01 bis 5 Gew.-% Triclosan und
(b) 45 bis 95 Gew.-% mindestens eines Polyalkylenglykols mit einer mittleren Molmasse (mittlerem Molekulargewicht) von 300 bis 1.000 g/mol enthält.

2. Vorrichtung nach Anspruch 1, wobei die Zusammensetzung (a) Triclosan in Mengen von 0,05 bis 3 Gew.-%. vorzugsweise 0,1 bis 2 Gew.-%, bevorzugt 0,1 bis 1 Gew.-%, besonders bevorzugt 0,15 bis 0,5 Gew.-%, ganz besonders bevorzugt 0,2 bis 0,4 Gew.-%, enthält, bezogen auf das Gewicht der Zusammensetzung.

3. Vorrichtung nach Anspruch 1 oder 2, wobei (b) das mindestens eine Polyalkylenglykol ein Polyethylenglykol ist.

4. Vorrichtung nach einem oder mehreren der vorangehenden Ansprüche, wobei das mindestens eine Polyalkylenglykol eine mittlere Molmasse (mittleres Molekulargewicht) von 325 bis 750 g/mol, bevorzugt 350 bis 600 g/mol, besonders bevorzugt 375 bis 450 g/mol, ganz besonders bevorzugt etwa 400 g/mol, aufweist.

5. Vorrichtung nach einem oder mehreren der vorangehenden Ansprüche, wobei die Zusammensetzung (b) das mindestens eine Polyol in Mengen von 50 bis 90 Gew.-%, vorzugsweise 55 bis 85 Gew.-%, bevorzugt 60 bis 85 Gew.-%, besonders bevorzugt 65 bis 80 Gew.-%, ganz besonders bevorzugt 70 bis 80 Gew.-%, enthält, bezogen auf das Gewicht der Zusammensetzung.

6. Vorrichtung nach einem oder mehreren der vorangehenden Ansprüche, wobei das Mengenverhältnis von (a) Triclosan einerseits zu (b) dem mindestens einen Polyol, vorzugsweise Polyethylenglykol, andererseits in der Zusammensetzung im Bereich von 1 : 10.000 bis 1 : 10, insbesondere 1 : 2.000 bis 1 : 20, vorzugsweise 1 : 1.000 bis 1 : 50, bevorzugt 1 : 500 bis 1 : 100, besonders bevorzugt 1 : 300 bis 1 : 200, liegt.

7. Vorrichtung nach einem oder mehreren der vorangehenden Ansprüche, wobei die Zusammensetzung in Form einer Lösung oder Suspension, insbesondere in Form einer wäßrigen Lösung oder wäßrigen Suspension, vorliegt und/oder wobei die Zusammensetzung Wasser enthält und/oder wobei die Zusammensetzung einen pH-Wert von 6 bis 8, insbesondere 6,5 bis 7,8, vorzugsweise 7 bis 7,5, aufweist.

8. Vorrichtung nach einem oder mehreren der vorangehenden Ansprüche, wobei die Zusammensetzung bei einer Temperatur von 20 °C eine Dichte von 1 bis 1,4 g/ml, insbesondere 1,025 bis 1,35 g/ml, vorzugsweise 1,05 bis 1,3 g/ml, bevorzugt 1,075 bis 1,25 g/ml, ganz besonders bevorzugt 1,1 bis 1,225 g/ml, aufweist.

9. Vorrichtung nach einem oder mehreren der vorangehenden Ansprüche, wobei die Zusammensetzung bei einer Temperatur von 20 °C eine dynamische Viskosität von 5 bis 1.000 mPas, insbesondere 5 bis 500 mPas, vorzugsweise 7,5 bis 100 mPas, bevorzugt 7,5 bis 50 mPas, besonders bevorzugt 10 bis 20 mPas, aufweist.

10. Vorrichtung nach einem oder mehreren der vorangehenden Ansprüche, enthaltend die Zusammensetzung in Form einer flüssigen Zubereitung, enthaltend in Kombination
(a) Triclosan in Mengen von 0,01 bis 5 Gew.-%, bevorzugt 0,05 bis 3 Gew.-%, vorzugsweise 0,1 bis 2 Gew.-%, bevorzugt 0,1 bis 1 Gew.-%, besonders bevorzugt 0,15 bis 0,5 Gew.-%, ganz besonders bevorzugt 0,2 bis 0,4 Gew.-%, bezogen auf das Gewicht der Zusammensetzung, und
(b) mindestens ein Polyalkylenglykol, vorzugsweise ein Polyethylenglykol, mit einer mittleren Molmasse (mittlerem Molekulargewicht) von 300 bis 1.000 g/mol, insbesondere 325 bis 750 g/mol, bevorzugt 350 bis 600 g/mol, besonders bevorzugt 375 bis 450 g/mol, ganz besonders bevorzugt etwa 400 g/mol, wobei das mindestens eine Polyalkylenglykol in Mengen von 45 bis 95 Gew.-%, insbesondere 50 bis 90 Gew.-%, vorzugsweise 55 bis 85 Gew.-%, bevorzugt 60 bis 85 Gew.-%, besonders bevorzugt 65 bis 80 Gew.-%, ganz besonders bevorzugt 70 bis 80 Gew.-%, vorliegt, bezogen auf das Gewicht der Zusammensetzung,
insbesondere wobei das Mengenverhältnis von (a) Triclosan einerseits zu (b) dem mindestens einen Polyol, vorzugsweise Polyethylenglykol, andererseits in der Zusammensetzung im Bereich von 1 : 10.000 bis 1 : 10, insbesondere 1 : 2.000 bis 1 : 20, vorzugsweise 1 : 1.000 bis 1 : 50, bevorzugt 1 : 500 bis 1 : 100, besonders bevorzugt 1 : 300 bis 1 : 200, liegt.

11. Vorrichtung nach einem oder mehreren der vorangehenden Ansprüche, aufweisend ein Behältnis, insbesondere eine Applikationsvorrichtung, vorzugsweise in Form einer sterilen Spritze, und einen Katheter, vorzugsweise einen Dauerkatheter, mit einer Aufnahmevorrichtung für die Zusammensetzung, insbesondere in Form einer Blockierungseinrichtung, vorzugsweise eines Blockierungsballons.

12. Vorrichtung nach Anspruch 11, wobei die Zusammensetzung dosierfertig und/oder applikationsfertig in dem Behältnis, insbesondere in Form einer sterilen Spritze, eingebracht vorliegt, insbesondere in Volumengrößen von 1 bis 50 ml, insbesondere 2 bis 30 ml, bevorzugt 5 bis 15 ml, besonders bevorzugt etwa 10 ml, vorliegt, je Behältnis bzw. Applikationseinheit (Dosiereinheit).

## Claims

1. A device for catheterization, in particular for the catheterization of the urinary bladder, containing a composition, in particular a pharmaceutical composition, in the form of a liquid preparation, the composition containing in combination, and each in effective, in particular pharmaceutically effective amounts, and each based on the weight of the composition, the following:
(a) 0.01 to 5% by weight triclosane, and
(b) 45 to 95% by weight at least of a polyalkylene glycol having a mean molar mass (mean molecular weight) of 300 to 1,000 g/mol.

2. The device according to claim 1, wherein the composition contains (a) triclosane at amounts of 0.05 to 3% by weight, preferably 0.1 to 2% by weight, preferably 0.1 to 1% by weight, particularly preferred 0.15 to 0.5% by weight, especially preferred 0.2 to 0.4% by weight, based on the weight of the composition.

3. The device according to claims 1 or 2, wherein (b) the at least one polyalkylene glycol is a polyethylene glycol.

4. The device according to one or more of the previous claims, wherein the at least one polyalkylene glycol has a mean molar mass (mean molecular weight) of 325 to 750 g/Moll, preferably 350 to 600 g/moll, particularly preferred 375 to 450 g/moll, especially preferred about 400 g/mol.

5. The device according to one or more of the previous claims, wherein the composition (b) contains the at least one polyol at amounts of 50 to 90% by weight, preferably 55 to 85% by weight, preferred 60 to 85% by weight, particularly preferred 65 to 80% by weight, especially preferred 70 to 80% by weight, based on the weight of the composition.

6. The device according to one or more of the previous claims, wherein the amount ratio of (a) triclosane on one hand, and (b) the at least one polyol, preferably polyethylene glycol on the other hand, in the composition is in the range of 1:10,000 to 1:10, in particular 1:2,000 to 1:20, preferably 1:1,000 to 1:50, preferred 1:500 to 1:100, especially preferred 1:300 to 1:200.

7. The device according to one or more of the previous claims, wherein the composition is present in the form of a solution or suspension, in particular in the form of an aqueous solution or aqueous suspension, and/or wherein the composition contains water, and/or wherein the composition has a pH of 6 to 8, in particular 6.5 to 7.8, preferably 7 to 7.5.

8. The device according to one or more of the previous claims, wherein at a temperature of 20°C, the composition has a density of 1 to 1.4 g/ml, in particular 1.025 to 1.35 g/ml, preferably 1.05 to 1.3 g/ml, preferred 1.075 to 1.25 g/ml, especially preferred 1.1 to 1.225 g/ml.

9. The device according to one or more of the previous claims, wherein at a temperature of 20°C, the composition has a dynamic viscosity of 5 to 1,000 mPas, in particular 5 to 500 mPas, preferably 7.5 to 100 mPas, preferred 7.5 to 50 mPas, especially preferred 10 to 20 mPas.

10. The device according to one or more of the previous claims, comprising the composition in the form of a liquid preparation, containing in combination
(a) triclosane in amounts of 0.01 to 5% by weight, preferably 0.05 to 3% by weight, preferably 0.1 to 2% by weight, preferably 0.1 to 1% by weight, particularly preferred 0.15 to 0.5% by weight, especially preferred 0.2 to 0.4% by weight, based on the weight of the composition, and
(b) at least one polyalkylene glycol, preferably a polyethylene glycol, having a mean molar mass (mean molecular weight) of 300 to 1,000 g/moll, in particular 325 to 750 g/moll, preferably 350 to 600 g/moll, particularly preferred 375 to 450 g/moll, especially preferred about 400 g/moll, wherein the at least one polyalkylene glycol is present in amounts of 45 to 95% by weight, in particular 50 to 90% by weight, preferably 55 to 85% by weight, preferred 60 to 85% by weight, particularly preferred 65 to 80% by weight, especially preferred 70 to 80% by weight, based on the weight of the composition,
in particular, wherein the amount ratio of (a) triclosane on one hand, to (b) the at least one polyol, preferably polyethylene glycol, on the other hand, in the composition is in the range of 1:10,000 to 1:10, in particular 1:2,000 to 1:20, preferably 1:1,000 to 1:50, preferred 1:500 to 1:100, particularly preferred 1:300 to 1:200.

11. The device according to one or more of the previous claims, having a container, in particular an application device, preferably in the form of a sterile syringe, and a catheter, preferably a permanent catheter, having a receiving device for the composition, in particular in the form of a blocking unit, preferably a blocking balloon.

12. The device according to claim 11, wherein the composition is present in an incorporated manner ready to dose, and/or ready to apply in a container, in particular in the form of a sterile syringe, in particular in volume sizes of 1 to 50 ml, in particular 2 to 30 ml, preferably 5 to 15 ml, particularly preferred about 10 ml, per container, or application unit (dosage unit), respectively.

## Revendications

1. Dispositif de cathétérisme, en particulier de cathétérisme de la vessie, contenant une composition, en particulier une composition pharmaceutique, sous la forme d'une préparation liquide, la composition contenant en combinaison et respectivement en quantités efficaces, en particulier pharmaceutiquement efficaces, et rapporté respectivement au poids de la composition
(a) 0,01 à 5 % en poids de triclosan et
(b) 45 à 95 % en poids d'au moins un polyalkylène glycol avec une masse molaire moyenne de 300 à 1000 g/mol.

2. Dispositif selon la revendication 1, dans lequel la composition (a) contient le triclosan en des quantités de 0,05 à 3 % en poids, de préférence de 0,1 à 2 % en poids, plus préférablement de 0,1 à 1 % en poids, encore plus préférablement de 0,15 à 0,5 % en poids, de façon particulièrement préférée de 0,2 à 0,4 % en poids, rapporté au poids de la composition.

3. Dispositif selon la revendication 1 ou 2, dans lequel (b) ledit au moins un polyalkylène glycol est un polyéthylène glycol.

4. Dispositif selon l'une ou plusieurs des revendications précédentes, dans lequel ledit au moins un polyalkylène glycol présente une masse molaire moyenne de 325 à 750 g/mol, de préférence de 350 à 600 g/mol, encore plus préférablement de 375 à 450 g/mol, de façon particulièrement préférée environ 400 g/mol.

5. Dispositif selon l'une ou plusieurs des revendications précédentes, dans lequel la composition (b) contient ledit au moins un polyol en des quantités de 50 à 90 % en poids, de préférence de 55 à 85 % en poids, plus préférablement de 60 à 85 % en poids, encore plus préférablement de 65 à 80 % en poids, de façon particulièrement préférée de 70 à 80 % en poids, rapporté au poids de la composition.

6. Dispositif selon l'une ou plusieurs des revendications précédentes, dans lequel le rapport quantitatif du (a) triclosan d'une part au (b), ledit au moins un polyol, de préférence du polyéthylène glycol, d'autre part, dans la composition, est compris dans l'intervalle de 1 : 10.000 à 1 : 10, en particulier de 1 : 2000 à 1 : 20, de préférence de 1 : 1000 à 1 : 50, encore plus préférablement de 1 : 500 à 1 : 100, de façon particulièrement préférée de 1 : 300 à 1 : 200.

7. Dispositif selon l'une ou plusieurs des revendications précédentes, dans lequel la composition se présente sous la forme d'une solution ou d'une suspension, en particulier sous la forme d'une solution aqueuse ou d'une suspension aqueuse, et/ou dans lequel la composition contient de l'eau et/ou dans lequel la composition présente un pH de 6 à 8, en particulier de 6,5 à 7,8, de préférence de 7 à 7,5.

8. Dispositif selon l'une ou plusieurs des revendications précédentes, dans lequel la composition présente à une température de 20 °C une densité de 1 à 1,4 g/ml, en particulier de 1,025 à 1,35 g/ml, de préférence de 1,05 à 1,3 g/ml, encore plus préférablement de 1, 075 à 1,25 g/ml, de façon particulièrement préférée de 1,1 à 1,225 g/ml.

9. Dispositif selon l'une ou plusieurs des revendications précédentes, dans lequel la composition présente à une température de 20 °C une viscosité dynamique de 5 à 1000 mPa.s, en particulier de 5 à 500 mPa.s, de préférence de 7,5 à 100 mPa.s, encore plus préférablement de 7,5 à 50 mPa.s, de façon particulièrement préférée de 10 à 20 mPa.s.

10. Dispositif selon l'une ou plusieurs des revendications précédentes, contenant la composition sous la forme d'une préparation liquide, contenant en combinaison
(a) du triclosan en des quantités de 0,01 à 5 % en poids, de préférence de 0,05 à 3 % en poids, plus préférablement de 0,1 à 2 % en poids, encore plus préférablement de 0,1 à 1 % en poids, de façon particulièrement préférée de 0,15 à 0,5 % en poids, en particulier de 0,2 à 0,4 % en poids, rapporté au poids de la composition, et
(b) au moins un polyalkylène glycol, de préférence un polyéthylène glycol avec une masse molaire moyenne de 300 à 1000 g/mol, en particulier de 325 à 750 g/mol, de préférence de 350 à 600 g/mol, encore plus préférablement de 375 à 450 g/mol, de façon particulièrement préférée d'environ 400 g/mol, sachant que ledit au moins un polyalkylène glycol est présent en des quantités de 45 à 95 % en poids, de préférence de 50 à 90 %, de préférence de 55 à 85 % en poids, plus préférablement de 60 à 85 % en poids, encore plus préférablement de 65 à 80 % en poids, de façon particulièrement préférée de 70 à 80 % en poids, rapporté au poids de la composition,
le rapport quantitatif dans la composition du (a) triclosan d'une part au (b), ledit au moins un polyol, de préférence du polyéthylène glycol, d'autre part, étant en particulier compris dans l'intervalle de 1 : 10.000 à 1 : 10, en particulier de 1 : 2000 à 1 : 20, de préférence de 1 : 1000 à 1 : 50, encore plus préférablement de 1 : 500 à 1 : 100, de façon particulièrement préférée de 1 : 300 à 1 : 200.

11. Dispositif selon l'une ou plusieurs des revendications précédentes, comportant un récipient, en particulier un dispositif d'application, de préférence sous la forme d'une seringue stérile, et un cathéter, de préférence un cathéter permanent, avec un dispositif de réception pour la composition, en particulier sous la forme d'un dispositif de blocage, de préférence d'un ballon de blocage.

12. Dispositif selon la revendication 11, dans lequel la composition se présente toute dosée et/ou prête à appliquer, conditionnée dans le récipient, en particulier sous la forme d'une seringue stérile, en particulier en volumes de 1 à 50 ml, plus particulièrement de 2 à 30 ml, de préférence de 5 à 15 ml, de façon particulièrement préférée d'environ 10 ml par récipient, respectivement par unité d'application (unité de dosage).
